# EUROPÄISCHE PATENTSCHRIFT

(11) **EP 2 869 855 B1**
(45) Veröffentlichungstag und Bekanntmachung des Hinweises auf die Patenterteilung: **04.11.2020**
(21) Anmeldenummer: 13747343.5
(22) Anmeldetag: 08.07.2013
(51) Int. Cl.: A61L 27/16, A61C 13/00, A61L 27/50, C08F 265/06, C08F 283/12, C08F 290/06, A61K 6/891, A61K 6/887

(54) **POLYMERISIERBARE MISCHUNGSZUSAMMENSETZUNG, VERWENDUNG DER MISCHUNGSZUSAMMENSETZUNG SOWIE EINE DENTALPROTHESE**
POLYMERIZABLE MIXTURE COMPOSITION, USE OF SAID MIXTURE COMPOSITION, AND A DENTAL PROSTHETIC
COMPOSITION DE MÉLANGE POLYMÉRISABLE, UTILISATION DE CETTE COMPOSITION DE MÉLANGE POLYMÉRISABLE ET PROTHÈSE DENTAIRE

(30) Priorität: 06.07.2012 DE 102012013514
(43) Veröffentlichungstag der Anmeldung: 13.05.2015
(73) Patentinhaber: Merz Dental GmbH, 24321 Lütjenburg (DE)
(72) Erfinder: PFLESSER, Sebastian, 24143 Kiel (DE); SEIFERT, Stefan, 24321 Lütjenburg (DE); ZIMEHL, Raif, 24235 Wendtorf (DE)
(74) Vertreter: Fish & Richardson P.C.
(86) Internationale Anmeldenummer: PCT/EP2013/002010
(87) Internationale Veröffentlichungsnummer: WO 2014/005727

(56) Entgegenhaltungen:
- WO-A1-01/12679
- DE-A1- 19 617 876
- GB-A- 2 173 506
- US-A- 3 887 669

## Beschreibung

Die vorliegende Erfindung betrifft eine polymerisierbare Mischungszusammensetzung, enthaltend eine liquide oder halbfeste Komponente A mit mindestens einem Monomeranteil und eine Feststoffkomponente B auf Polymethylmethacryalat (PMMA)-Basis mit einem Füllstoff und/oder Zusatzstoffen, die Verwendung der Mischungszusammensetzung sowie eine Dentalprothese, hergestellt aus der Mischungszusammensetzung.

Herkömmliche Kunststoffe für Formteile, insbesondere handelsübliche Prothesenbasiskunststoffe, auf Polymethylmethacrylat-Basis im Dentalbereich, weisen auf Grund der physikalischen Eigenschaften von Polymethylmethacrylat (PMMA) eine nur sehr geringe Brucharbeit und eine mittlere Bruchzähigkeit auf.

PMMA und seine handelsüblichen Copolymere besitzen eine recht hohe Härte und Sprödigkeit bzw. Brüchigkeit, was bei einer Belastung zunächst dazu führt, dass eine relativ hohe Kraft überwunden muss, um einen Materialbruch über Rissfortpflanzung herbeizuführen. Dies führt zu einem mittleren Wert bei der Bruchzähigkeit, welche angibt, wieviel Energie zur Rissfortpflanzung aufgewendet werden muss. Ist diese Energie jedoch erst einmal überwunden, setzt sich der Riss augenblicklich durch das gesamte Material fort und dieses zerspringt. Beispielsweise wäre dann eine Dentalprothese nicht mehr nutzbar und müsste, sofern möglich, zur Reparatur.

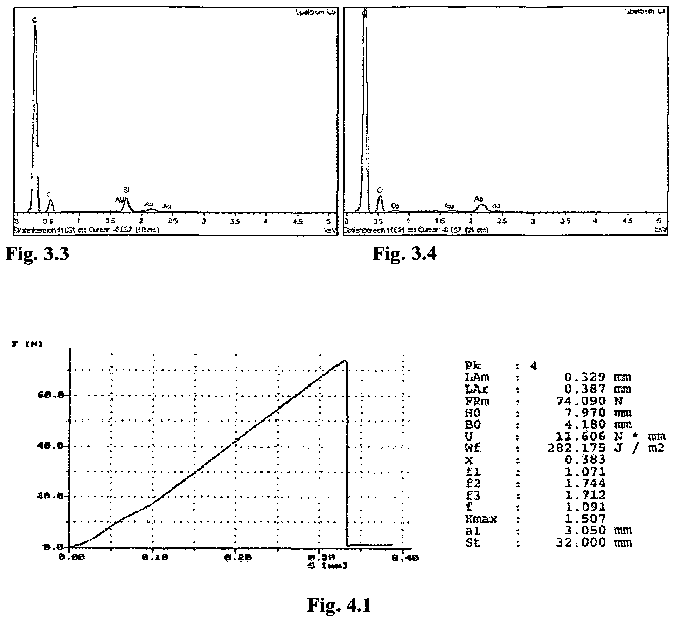

Auf Grund dieses Bruchverhaltens von PMMA ergibt sich ein sehr niedriger Wert bei der Brucharbeit. Diese gibt an, wieviel Energie je Flächeneinheit nötig ist, um einen vorgeschädigten, genormten Probekörper zu zerbrechen. Da die aufgewendete Energie bei einem Splitterbruch schlagartig auf 0 sinkt, ist diese Arbeit (Brucharbeit, W_{f}) sehr gering, vgl. die grafische Darstellung in Fig. 4.1.

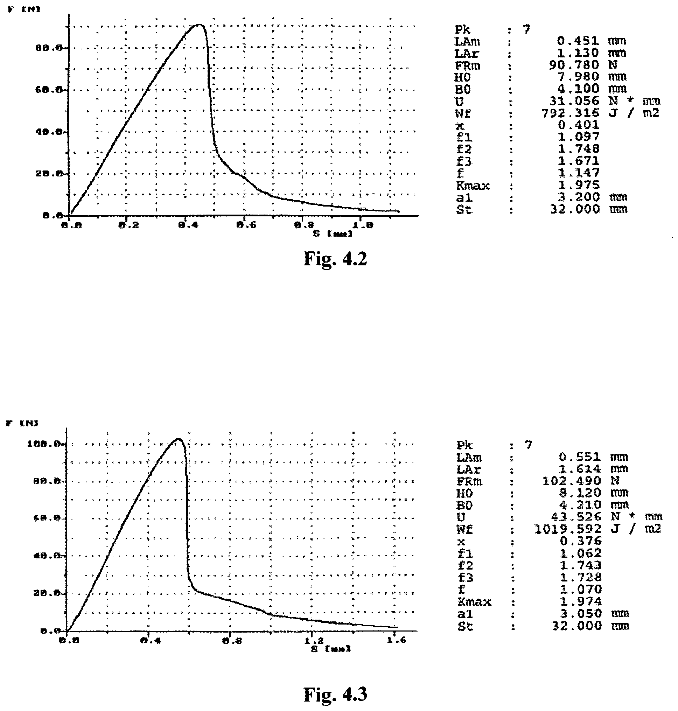

Bei einem bruchzähen Material hingegen setzt die Rissfortpflanzung zwar bei einem Energiemaximum ein, definiert als Bruchzähigkeit, (Kₘₐₓ), dieser Riss zieht sich dann aber nur langsam durch den Probekörper, da das Material auf Grund einer Modifikation eine signifikant erhöhte Zähigkeit besitzt und so in der Lage ist, die an der Rissspitze wirkenden Kräfte zu dissipieren. Es ergibt sich, dass ein bruchzähes Material bedeutend mehr Kraft / Fläche vertragen kann, bevor der Riss die gegenüberliegende Seite des Probekörpers/Formteils erreicht und es ggf. sogar zerbricht; vgl. die grafische Darstellung in Fig. 4.2 und 4.3, wobei die Skalierung der Graphen zu beachten ist.

Zur Herstellung von individuellen Formteilen wie z. B. zahntechnischen Prothesen sind am Markt verschiedene Verfahren mit den dazugehörigen Materialien vorhanden. Einen immer größer werdenden Marktanteil gewinnt hier das Gießverfahren mit autopolymerisierenden Kunststoffen auf Basis von PMMA. Hierbei handelt es sich üblicherweise um ein 2-Komponenten-System, welches zum einen aus einer flüssigen Komponente, überwiegend bestehend aus einem monofunktionellen polymerisierbaren Monomer, insbesondere Methylmethacrylat, einem Vernetzer, insbesondere difunktionelle Alkyldimethacrylate, z.B. Ethylenglykoldimethacrylat oder 1,4-Butandioldimethacrylat, ggf. Additive zur Regulierung der Molmasse der resultierenden Polymere, z.B. Thiole, sowie geringe Konzentrationen eines entsprechenden Initiatorsystems, z.B. Peroxid/Amin-Systeme oder Systeme auf Basis von Barbitursäurederivaten, Stabilisatoren für die Lagerung, z.B. Hydrochinonmonomethylether und/oder Farb- oder mikrobiell wirksame Verbindungen besteht.

Zum anderen ist eine feste, bevorzugt pulverförmige Komponente vorhanden, welche überwiegend Polymethylmethacrylat und dessen Copolymere beliebiger Zusammensetzungen sowie in beliebigen Mischungsverhältnissen, insbesondere in Perlform umfasst. Optional werden zusätzlich Füllstoffe, z.B. Silikate oder Apatite, Farbstoffe, z.B. Azokondensationsprodukte, und Pigmente, überwiegend Eisen- und Titanoxide, sowie Modifikatoren, z.B. zur Einstellung einer Röntgenopazität oder mikrobiologisch wirksame Materialien und weitere Initiatorkomponenten zur festen (zweiten) Komponente hinzugefügt.

Die beiden genannten Komponenten werden üblicherweise in einem festgelegten Verhältnis miteinander vermischt, sodass hieraus eine fließfähige Mischung entsteht. Auf Grund der Tatsache, dass sich PMMA sowie die meisten seiner unvernetzten Copolymere in seinem Monomer bzw. MMA löst bzw. zumindest quellbar sind, steigt die Konsistenz der Mischung mit fortschreitender Zeit stetig an und wird alsbald in Form, beispielsweise in eine entsprechend vorbereitete Dentalform, gegossen. Hierin teigt die Mischung immer weiter an, wobei die Initiatorkomponenten zeitnah die Polymerisation starten.

Die Offenlegungsschrift DE 196 17 876 A1 betrifft ein polymerisierbares Dentalmaterial, welches mindestens ein polymerisierbares Monomer und als Schlagfestigkeitsmodifikator ein Polysiloxan-Pfropfcopolymerisat, welches einen Kern aus elastomerem Polysiloxan und eine Hülle aus nicht-elastomerem Polymer aufweist, und/oder ein Polysiloxan mit (Meth)acrylGruppen und einen Vernetzer, die als Dispersionen vorliegen.

GB 2 173 506 A1 beschreibt Mischungen von Siloxanpolymeren, die eine steife Leiterstruktur aufweisen und die über eine polymerisierbare funktionelle ethylenische Gruppe sowie über eine hydrophobe Gruppe in der Seitenkette des gleichen Moleküls verfügen, und die eine Si-O-Bindung in der Hauptkette besitzen, mit einem copolymerisierbaren Monomeren als Mischungspartner.

US 3,887,669 B betrifft ein Verfahren zur Herstellung eines Verbunderzeugnisses, das einen Teil aus Acrylharz enthält, haftend an einem Teil, welcher aus einem Siliconelastomeren hergestellt ist. Damit wird erreicht, auf direktem Wege eine Siliconelastomer/Acrylharz-Bindung herzustellen.

WO 01/12679 A1 beschreibt eine im sichtbaren Licht aushärtende Kunststoffzusammensetzung, die für formgebende Verfahren, wie die Mikrophotoverfestigung, oder für die Herstellung von Dentalprodukten geeignet ist, wobei Methylmethacrylatgruppen-haltige Monomere als potentieller Bestandteil einer Harzmasse genannt sind, die unter dem Einfluss von sichtbarem Licht oder im Rahmen einer radikalischen Polymerisationsreaktion aushärten. Feststoffe werden durch anorganische Verbindungen oder Fasern repräsentiert.

Die Druckschriften EP 1 702 633 A2 bzw. DE 10 2005 012 825 A1 offenbaren in diesem Zusammenhang ein bruchzähes autopolymerisierendes "High-Impact-Material" ("PalaXpress Ultra", Heraeus Kulzer). Darin wird auch der Einsatz von elastisch modifizierten Perlpolymerisaten bei autopolymerisierenden 2-Komponenten-Dentalprothesenbasismaterialien beschrieben, um die gemäß DIN EN ISO-Norm geforderten Werte hinsichtlich der Bruchzähigkeit und Brucharbeit zu erreichen. Die elastische Modifizierung der Perlpolymerisate erfolgt dabei durch Kern-Schale-Partikel jeglicher Art, z.B. durch klassische Kern-Hülle-Teilchen (mit elastischem Kern), Multicore-Partikel und mehrere Kern-Hülle-Teilchen in fester Matrix oder durch interpenetrierende Netzwerke aus elastischer und fester Phase innerhalb der Perlpolymerisate.

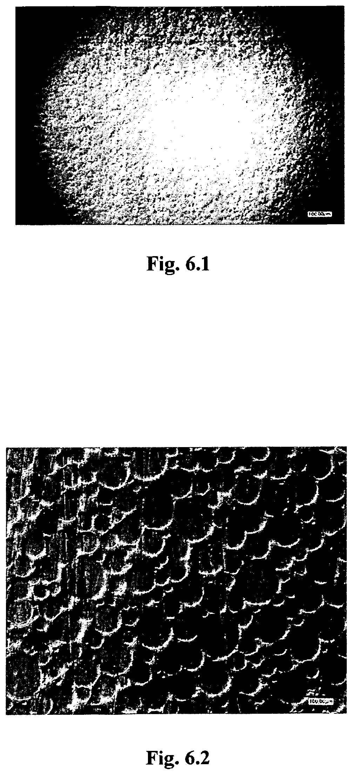

Jedoch wird hierbei nachteilig ausschließlich die Polymerkomponente modifiziert. Nach dem Vermischen und Polymerisieren mit der Monomerkomponente ergibt sich eine Materialstruktur aus kreisrunden, bruchzäh modifizierten Polymerperlen (vgl. Abb. 6.1 und 6.2), die durch die polymerisierte Monomermatrix verbunden sind. Da die Monomermatrix aber nicht modifiziert wurde, besitzt diese immer noch die hohe Sprödigkeit bzw. Brüchigkeit und somit eine geringe Bruchzähigkeit. Zudem ist bei einem derartigen Polymerblend nicht gewährleistet, dass die modifizierten Partikel homogen verteilt sind. Derartige intrinsische Inhomogenitäten führen in ungünstigen Fällen zu unerwünschten Sollbruchstellen.

Die Aufgabe vorliegender Erfindung liegt daher in der Angabe einer gießfähigen, autopolymerisierenden Mischungszusammensetzung, welche eine gegenüber herkömmlichen PMMA-Materialien signifikant erhöhte Brucharbeit und Bruchzähigkeit, vorzugsweise entsprechend der Anforderungen und Definitionen nach Produktnorm DIN EN ISO 20795-1:2008) aufweist und die genannten Nachteile mittels inhärenter Material- bzw. Kompositionseigenschaften vermeidet. Ferner besteht die Aufgabe der Erfindung in der Bereitstellung einer geeigneten Verwendung einer der derartigen Zusammensetzung sowie eines entsprechenden Formkörpers.

Diese Aufgabe wird durch die Merkmale der unabhängigen bzw. nebengeordneten Ansprüche 1, 9 und 11 gelöst.

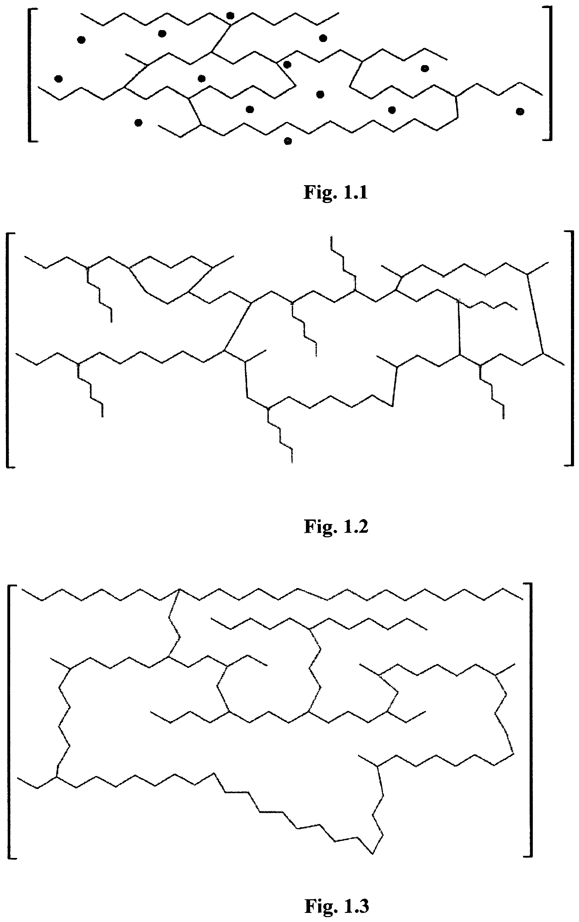

Ein wesentlicher Aspekt der Erfindung besteht in der Bereitstellung einer polymerisierbaren Mischungszusammensetzung enthaltend eine liquide oder halbfeste Komponente A mit mindestens einem Monomeranteil, und eine, bevorzugt pulverförmige, Feststoffkomponente B auf Polymethylmethacryalat (PMMA)-Basis mit einem Füllstoff und/oder Zusatzstoffen, wobei die liquide oder halbfeste Komponente A zusätzlich mindestens eine den Monomeranteil modifizierende oligomere oder polymere Verbindung aufweist, welche mit dem Monomeranteil mischbar ist. Mit anderen Worten wird hier nicht die feste Polymerkomponente hinsichtlich der Brucheigenschaften des nach der Polymerisation erhaltenen Formkörpers modifiziert, sondern vorteilhaft der flüssige Monomeranteil in Komponente A. Der Monomermischung wird ein beispielsweise lineares Poly(organo)siloxan zugesetzt, welches sich mit der flüssigen Komponente A mischt und auch später keine Phasentrennung hervorruft und in das System mit einpolymerisiert werden kann. Dies kann zum Einen auf physikalischem Wege erfolgen, d.h. durch Einlagerung sog. äußerer Modifikatoren, vgl. beispielsweise Fig. 1.1, oder chemisch durch die Ausbildung kovalenter Bindungen und Entstehung von Copolymeren als sog. innere Modifikatoren, vgl. Fig. 1.2 und 1.3.

Die pulverförmige Feststoffkomponente B ist dabei durch die flüssige oder halbfeste, d.h. viskose oder ggf. pastöse Komponente A enthaltend das Monomer mindestens benetzbar, bevorzugt darin dispergierbar, insbesondere bevorzugt quellbar und im Idealfalle, besonders bevorzugt, in der flüssigen oder halbfesten, pastösen Komponente A ganz löslich.

Bevorzugt enthält die Feststoffkomponente B und/oder die liquide oder halbfeste Komponente A Initiatorkomponenten, die bei einer Vermischung die Polymerisation selbstständig auslösen.

Vorzugsweise liegt der Anteil der oligomeren oder polymeren Verbindung in der liquiden oder halbfesten Komponente A in einem Bereich zwischen 0,1 und 50 Gew.-% bzw. zwischen 0,1 und 30 Gew.-%, bevorzugt zwischen 0,5 und 25 Gew.-% und besonders bevorzugt zwischen 1 und 20 Gew.-%.

Erfindungsgemäß ist wenigstens eine der oligomeren oder polymeren Verbindung ein Polyorganosiloxan.

Erfindungsgemäß ist die Polymerverbindung bzw. das wenigstens eine Polyorganosiloxan ausgewählt aus der Gruppe bestehend aus Polydimethylsiloxanen (PDMS), Polydiphenylsiloxanen (PDPS), Polymethylphenylsiloxanen (PMPS) und/oder Mischungen daraus.

Das Polyorganosiloxan ist bevorzugt mit dem Monomeranteil der liquiden oder halbfesten Komponente A homogen mischbar und/oder weist bevorzugt eine lineare Kettenstruktur auf.

Dabei liegt die mittlere Anzahl (n) der linearen Polyorganosiloxangruppen vorzugsweise in einem Bereich zwischen 2 und 500 bzw. zwischen 2 und 200, bevorzugt zwischen 5 und 200 bzw. zwischen 5 und 150, besonders bevorzugt im Bereich zwischen 7 und 75 bzw. zwischen 7 und 40.

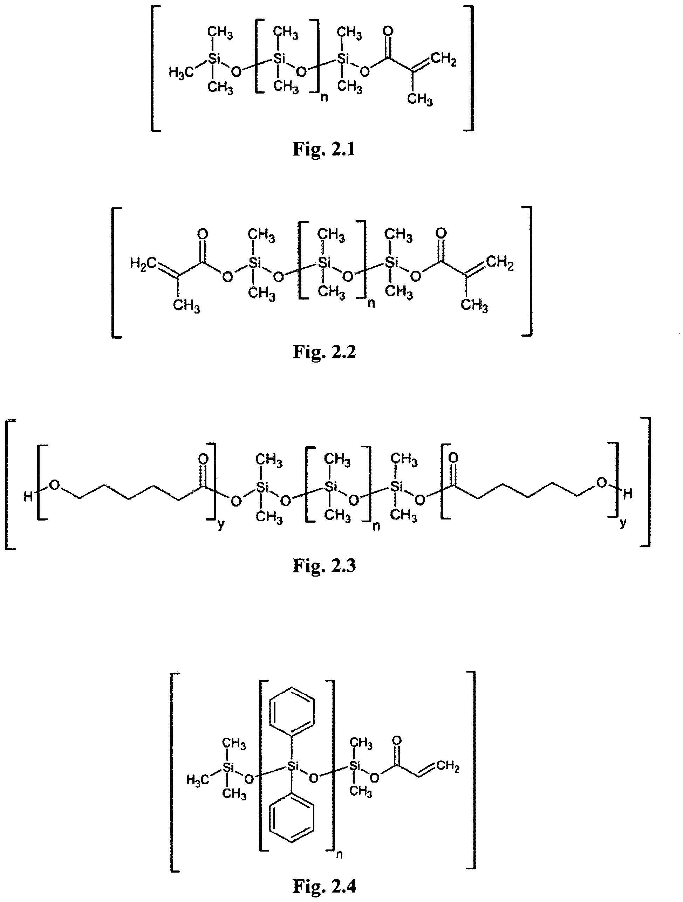

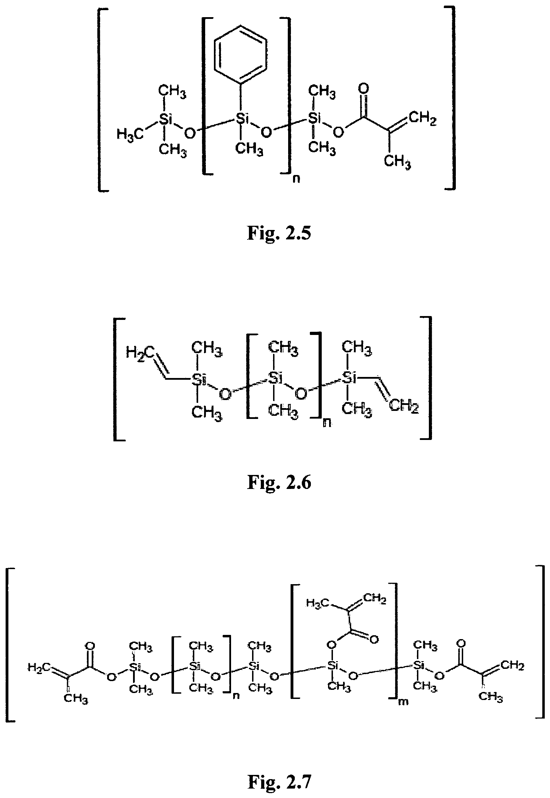

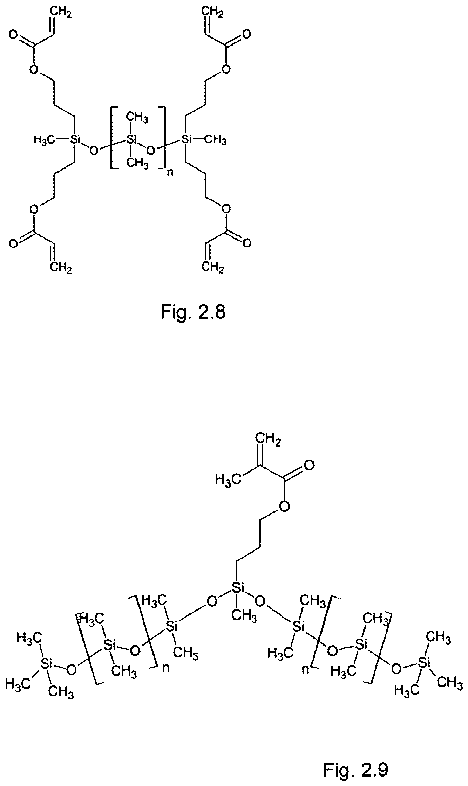

Weiter bevorzugt ist die Polyorganosiloxan-Kettenstruktur modifiziert, insbesondere ist diese mittels Substituenten, chemisch modifiziert. Bei dieser Modifizierung handelt es sich bevorzugt um Polymere, die sich mit PMMA-Systemen vertragen, d.h. mit diesen mischbar und/oder umsetzbar sind, z.B. (Poly-) Caprolactone, vgl. Fig. 2.3, besonders bevorzugt um ungesättigte Gruppen wie z.B. Vinyl- (vgl. Fig. 2.6), Allyl-, und (Meth-)Acrylgruppen (vgl. Fig. 2.2, 2.8 und 2.9) und ganz besonders bevorzugt um eine (einseitige) Kettenterminierung mit einer (Meth)Acrylgruppe gemäß Fig 2.1, Fig. 2.4 und Fig. 2.5.

In einer bevorzugten Ausführungsform weist die polymerisierte Mischungszusammensetzung bzw. der Kunststoffformkörper eine Bruchzähigkeit (Kₘₐₓ) ≥ 1,9 kJ/m^{1/2} und eine Brucharbeit ≥ 900 J/m² auf bzw. der Kunststoffformkörper gegenüber einer unmodifizierten und polymerisierten Mischungszusammensetzung sowohl eine erhöhte Bruchzähigkeit (Kₘₐₓ) als auch erhöhte Brucharbeit (W_{f}).

In einer erfindungsgemäßen Verwendung einer o.g. polymerisierbaren Mischungszusammensetzung zur Herstellung eines Kunststoffformkörpers weist die polymerisierte Mischungszusammensetzung bzw. der Kunststoffformkörper eine Bruchzähigkeit (Kₘₐₓ) ≥ 1,9 kJ/m^{1/2} und eine Brucharbeit ≥ 900 J/m² gem. DIN EN ISO 20795-1:2008 auf.

Der so hergestellte Kunststoffformkörper wird vorzugsweise als Dentalprothesenkörper eingesetzt und genügt in einer Ausführungsform den Mindestvorgaben gemäß ISO für High-Impact-Kunststoffe.

Bevorzugt wird dabei die polymerisierbare Mischungszusammensetzung als Reparaturwerkstoff für derartige Formkörper oder dgl. eingesetzt. Dies umfasst insbesondere auch die Verwendung als Reparaturmaterial für Heißpolymerisate.

Ferner wird erfindungsgemäß eine Dentalprothese bereitgestellt, welche aus einer der oben definierten polymerisierbaren Mischungszusammensetzungen hergestellt ist.

Die nachfolgenden Beispiele und Figuren sollen die Erfindung und ihre Vorteile veranschaulichen. Dabei zeigen:
- Fig. 1.1: ein erfindungsgemäßes PMMA-Netzwerk mit äußerem Modifikator (z.B. W35);
- Fig. 1.2: ein erfindungsgemäßes PMMA-Netzwerk amit innerem Modifikator (z.B. Fluid-MA-15M);
- Fig. 1.3: ein erfindungsgemäßes PMMA-Netzwerk mit vernetzendem Modifikator (z.B. Fluid-MA-40D);
- Fig. 2.: Beispiele erfindungsgemäßer Poly(organo)siloxane:
- Fig. 2.1: Mono-MA-funktionelles PDMS (z.B. Fluid-MA-15M);
- Fig. 2.2: Di-MA-funktionelles PDMS (z.B. Fluid-MA-40D);
- Fig. 2.3: Polycaprolactonmodifiziertes PDMS (z.B. W35);
- Fig. 2.4: Mono-AC-funktionelles PDPS;
- Fig. 2.5: Mono-MA-funktionelles PMPS;
- Fig. 2.6: Di-Vinyl-funktionelles PDMS;
- Fig. 2.7: Kammartiges multi-MA-funktionelles PDMS;
- Fig. 2.8: Endständig multiacryliertes PDMS;
- Fig. 2.9: Mittelständig methacryliertes PDMS;
- Fig. 3.1: REM-Aufnahme einer Polymerstruktur eines Dentalkunststoffes mit zwei EDX-Messpunkten (vgl. Fig. 3.3 und Fig. 3.4);
- Fig. 3.2: Silizium-selektives Elementmapping (EDX) im REM eines erfindungsgemäßen Formteils;
- Fig. 3.3: EDX-Spektrum der die Perlen umgebenden Matrix;
- Fig. 3.4: EDX-Spektrum des Perlpolymerisats;
- Fig. 4.1: Kraftdiagramm eines Polymerformteils aus dem Stand der Technik (Standardmischung);
- Fig. 4.2: Kraftdiagramm eines erfindungsgemäßen Polymerformteils (Beispiel 4, s.u.);
- Fig. 4.3: Kraftdiagramm eines erfindungsgemäßen Polymerformteils (Beispiel 2, s.u);
- Fig. 5.1: IR-Spektrum von Methylmethacrylat (Sigma-Aldrich);
- Fig. 5.2: IR-Spektrum von Polydimethylsiloxan (Sigma-Aldrich);
- Fig. 6.1: Struktur eines autopolymerisierten PMMA-Kunststoffes (100-fache Vergrößerung) und
- Fig. 6.2: Struktur eines autopolymerisierten PMMA-Kunststoffes (600-fache Vergrößerung).

### Beispiele

### Zusammensetzung einer allgemeinen Polymerkomponente:

97 g MW134 (Polymethylmethacrylat-Copolymer, Fa. Evonik) und 3 g 1-Benzyl-5-Phenyl-barbitursäure.

### Zusammensetzung einer Monomermischung (Standardmischung) aus dem Stand der Technik:

95,6 g Methylmethacrylat, 4 g 1,4-Butandioldimethacrylat, 0,2 g Dilauryldimethylammoniumchlorid und 0,2 g Kupfernaphthenat-Konzentrat werden zusammen gegeben und bilden nach dem Auflösen des Chlorids eine glasklare, homogene Lösung.
Beispiel 1 einer erfindungsgemäßen Zusammensetzung einer (Monomer)-Komponente A: 93,6 g Methylmethacrylat, 4 g 1,4-Butandioldimethacrylat, 2 g Fluid-MA-15M, 0,2 g Dilauryldimethylammoniumchlorid und 0,2 g Kupfernaphthenat-Konzentrat werden zusammen gegeben und bilden nach dem Auflösen des Chlorids eine glasklare, homogene Lösung. Beim Fluid-MA-15M handelt es sich um ein dünnflüssiges, klares, lineares Polydimethylsiloxan mit einer mittleren Kettenlänge von n=15 und einer einseitigen endständigen Methacrylgruppe (vgl. Fig. 2.1).
Beispiel 2 einer erfindungsgemäßen Zusammensetzung einer (Monomer)-Komponente A: 80,6 g Methylmethacrylat, 4 g 1,4-Butandioldimethacrylat, 15 g Fluid-MA-15M, 0,2 g Dilauryldimethylammoniumchlorid und 0,2 g Kupfernaphthenat-Konzentrat werden zusammen gegeben und bilden nach dem Auflösen des Chlorids eine glasklare, homogene Lösung.
Beispiel 3 einer erfindungsgemäßen Zusammensetzung einer (Monomer)-Komponente A: 90,6 g Methylmethacrylat, 4 g 1,4-Butandioldimethacrylat, 5 g W35, 0,2 g Dilauryldimethylammoniumchlorid und 0,2 g Kupfernaphthenat-Konzentrat werden zusammen gegeben und bilden nach dem Auflösen des Chlorids sowie des W35 eine glasklare, homogene Lösung. Beim W35 handelt es sich um ein lineares Polydimethylsiloxan mit einer mittleren Kettenlänge n=40, dessen Kettenenden mit einem Polycaprolacton modifiziert wurden (mittlere Kettenlänge y=15 pro Seite). Es besitzt eine wachsartige Struktur, löst sich aber gut in MMA und dessen Co-Monomeren (vgl. Fig. 2.3).
Beispiel 4 einer erfindungsgemäßen Zusammensetzung einer (Monomer)-Komponente A: 85,4 g Methylmethacrylat, 4 g Butandioldimethacrylat, 10 g Fluid-MA-40D, 0,2 g Dilaurylidimethylammoniumchlorid und 0,2 g Kupfenaphthenat-Konzentrat werden zusammen gegeben und bilden nach dem Auflösen des Chlorids eine glasklare, homogene Lösung. Beim Fluid-MA-40D handelt es sich um ein dünnflüssiges, klares, lineares Polydimethylsiloxan mit einer mittleren Kettenlänge von n=40 und einer beidseitigen Endgruppenmodifizierung mit je einer Methacrylgruppe (vgl. Fig. 2.2).
Beispiel 5 einer erfindungsgemäßen Zusammensetzung einer (Monomer)-Komponente A: 91,6 g Methylmethacrylat, 4 g 1,4-Butandioldimethacrylat, 4 g MD-M-2520Mu, 0,2 g Dilauryldimethylammoniumchlorid, 0,2 g Kupfernaphthenat-Konzentrat werden zusammen gegeben und bilden nach dem Auflösen des Chlorids eine glasklare, homogene Lösung. Beim MD-M-2520Mu handelt es sich um ein flüssiges, klares, lineares PDMS mit einer mittleren Kettenlänge von n=35 und einer mehrfach acrylierten beidseitigen Kettenterminierung (vgl. Fig. 2.8).
Beispiel 6: Polymerisation und Prüfung der Zusammensetzungen aus den Beispielen 1-4 sowie der Standardmischung:
   20g der Polymerkomponente B werden mit je 14ml einer Monomerkomponente A angerührt, in eine rechteckige Form gegossen und bei 2 bar und 50°C über eine Zeit von 15 Minuten in einem Dental-Drucktopf polymerisiert. Die Platte aus der Standardmischung ist nahezu glasklar, während die modifizierten Platten eine deutliche, aber homogene Opazität aufweisen, wobei diese eine derartige Intensität aufweisen, dass die Materialien in den handelsüblichen Dentalfarben eingestellt werden können.

Die Prüfung der physikalischen Werte bzgl. Brucharbeit (W_{f}) und Bruchzähigkeit (K) sowie Biegebruchfestigkeit (BF) und Biegemodul (BM) erfolgt gemäß DIN EN ISO 20795-1:2008, vgl. Tabelle 1.

**Tabelle 1**

| | | | | |
|---|---|---|---|---|
| Standardmischung | BM= 2571 ± 54 | BF= 83,8 ± 2,3 | W_{f}=295 ± 16 | K= 1,53 ± 0,09 |
| 1. Mischung A | BM= 2511 ± 70 | BF= 79,9 ± 2,7 | Wf = 848 ± 28 | K= 2,44 ± 0,06 |
| 2. Mischung A | BM= 1936 ± 53 | BF= 51,7 ± 2,6 | Wf = 941 ± 88 | K= 1,92 ± 0,09 |
| 3. Mischunq A | BM= 2481 ± 100 | BF= 114,6 ± 1,3 | Wf = 592 ± 70 | K= 1,87 ± 0,06 |
| 4. Mischung A | BM= 2198 ±. 94 | BF= 74,7 ± 12,3 | Wf = 710 ± 119 | K= 1,78 ± 0,11 |
| 5. Mischung A | | | Wf = 696 ± 22 | K= 1,85 ± 0,10 |

Es zeigt sich, dass Zusätze von modifizierten Polyorganosiloxanen zur Monomermischung eine signifikante Erhöhung bezüglich der von der Norm geforderten physikalischen Werte hinsichtlich der Gesamtbrucharbeit sowie der Bruchzähigkeit aufweisen und die erfindungsgemäße Mischung A aus Beispiel 2 besonders vorteilhaft die Grenzwerte für hervorzuhebende Materialeigenschaften mit W_{f} ≥ 900 und K ≥ 1,9 erfüllt und die Mischung 1 nach Beispiel 1 bei ungefährer Beibehaltung von BM und BF diese Anforderungen näherungsweise erreicht.

Der Hauptanteil der Monomerkomponente A ist mit > 80% Methylmethacrylat und gewährleistet somit eine Verträglichkeit/Löslichkeit mit bzw. von der Polymerkomponente B und, beispielsweise, zu den während der Verarbeitung eingegliederten künstlichen Zähnen oder den Kontaktstellen mit bereits polymerisierten Materialien beim Einsatz als Reparaturwerkstoff. In der Verwendung und insbesondere im Herstellprozess ist die Mischung von flüssigen oder halbfesten Komponenten bedeutend einfacher als das Lösen von Feststoffen, was eine signifikante Verbesserung in der Verwendung gegenüber herkömmlichen Materialien darstellt.

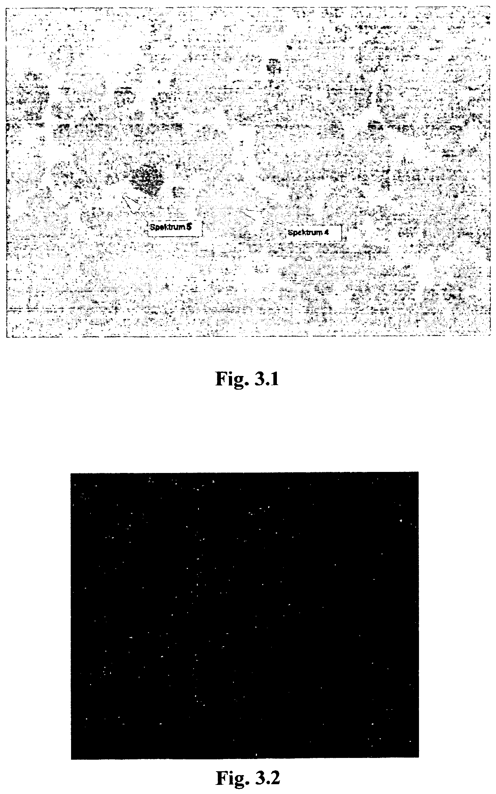

Bei der Modifizierung der Monomerkomponente mit flüssigen Materialien ist eine homogene Verteilung der Zusatzstoffe gewährleistet, vgl. Fig. 3.2, während bei einer herkömmlichen Feststoff-Verteilung in Komponente B diese nicht einwandfrei bzw. vollständig homogen gegeben ist.

Auf Grund der homogenen Verteilung der Siloxane in der Monomerkomponente A sind diese auch besonders vorteilhaft später homogen im Werkstück verteilt und sorgen für gleichmäßige physikalische Eigenschaften, was bei Feststoffen auf Grund von möglicher Agglomeratbildung oder Sedimentierung der pulverförmigen Stoffe durch unterschiedliche Korngröße und/oder Dichte nicht gewährleistet ist.

Durch den Zusatz von Poly(organo)siloxanen wird das spätere Werkstück darüber hinaus in seiner Gesamtheit hydrophober eingestellt und somit die Wasseraufnahme und -löslichkeit herabgesetzt. Die geringere Wasseraufnahme führt vorteilhaft dazu, dass sich weniger Bakterien bzw. Plaque auf dem Werkstück, besonders vorteilhaft bei Verarbeitung des Werkstücks zur späteren Dentalprothese, festsetzen können. Ferner lässt sich durch den Einsatz der Polysiloxane in der Zusammensetzung die Materialoberfläche besser polieren. Dies führt vorteilhaft zu einer besseren Oberflächengüte und besseren Resistenz gegenüber Ablagerungen.

Auf Grund der bevorzugt vorhandenen ungesättigten Gruppen, insbesondere (Meth)Acrylgruppe(n) werden die Polysiloxane chemisch in das Polymernetzwerk eingebunden und somit ein späteres Ausdiffundieren vorteilhaft verhindert.

Durch die signifikante Verbesserung der nötigen physikalischen Eigenschaften des Formteils und die vereinfachte Handhabbarkeit der Zusammensetzung über die ausschließliche Modifizierung der Monomerkomponente wird darüber hinaus eine neue Klasse von Basismaterial zur Prothesenherstellung bereitgestellt.

Darüberhinaus wird durch den Zusatz der Polysiloxane zur Monomerkomponente der prozentuale Anteil an polymerisierbaren Gruppen reduziert und somit der polymerisationsbedingte Gesamtvolumenschrumpf des Werkstückes vorteilhaft reduziert. Werden die Poly(organo)siloxane mit Phenylgruppen modifiziert, wird deren Brechungsindex weiter an den von PMMA angeglichen, sodass die Transparenz gegenüber herkömmlichen Poly(organo)siloxanen signifikant zunimmt und somit ein weiterer Vorteil gegenüber der Standard-Modifizierung entsteht (vgl. Fig. 2.4 und 2.5).

Werden Poly(organo)siloxane eingesetzt, die zudem weitere ungesättigte Gruppen entlang der Siloxan-Kette tragen (vgl. Fig. 2.6) und somit eine kammähnliche Struktur aufweisen, wird die Verträglichkeit mit dem System signifikant verbessert und es können höhere Konzentrationen eingesetzt werden um die physikalischen Werte weiter zu optimieren.

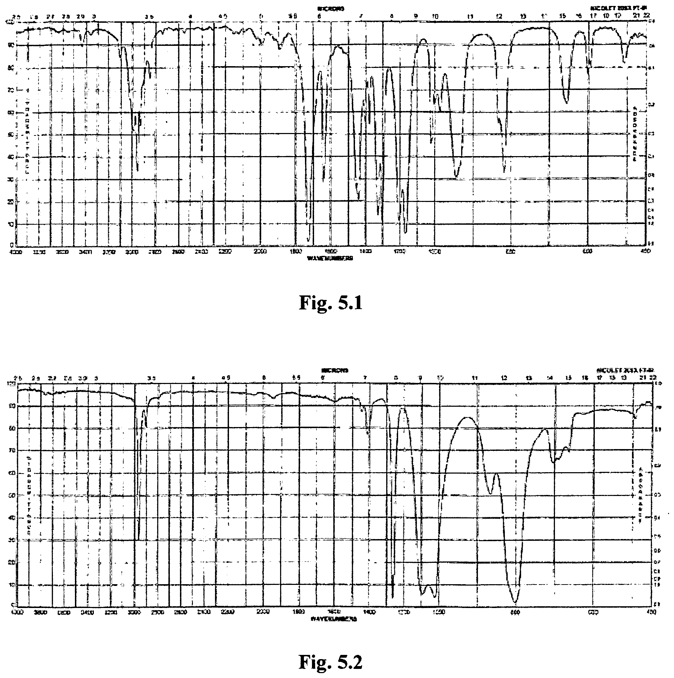

Die IR-Spektren nach Fig. 5.1 und Fig. 5.2 zeigen signifikante Unterschiede zu den Spektren herkömmlicher Materialien, z.B. 1070: antisymmetrische Streckschwingung Si-O-Si, 800 und 840: Mono- und dimethylsiloxan-Gruppen; vgl. Infrared spectra and structure of thin polydimethylsiloxane films, E.A. Romaneko und B.V. Tkachuk, sodass der Zusatz von Poly(organo)siloxanen in der Monomerkomponente über IR-Spektroskopie zur chemischen Identifizierung herangezogen werden kann.

Im polymerisierten Material sind Poly(organo)siloxane durch REM-EDX-Aufnahmen identifizierbar, vgl. Fig. 3.1 bis Fig. 3.4, da auf Grund des Einsatzes der Modifikatoren in der Monomerkomponente diese in den die Perlen umgebenden Zwischenbereichen leicht detektierbar sind.

Sämtliche in vorliegenden Unterlagen genannten Merkmale werden als erfindungswesentlich beansprucht.

## Patentansprüche

1. Polymerisierbare Mischungszusammensetzung enthaltend eine liquide oder halbfeste Komponente A mit mindestens einem Monomeranteil, und eine Feststoffkomponente B auf Polymethylmethacryalat (PMMA)-Basis mit einem Füllstoff und/oder Zusatzstoffen, wobei die liquide oder halbfeste Komponente A zusätzlich mindestens ein den Monomeranteil modifizierende oligomere oder polymeres Polyorganosiloxan aufweist, welches mit dem Monomeranteil mischbar ist, ausgewählt aus der Gruppe bestehend aus Polydimethylsiloxanen (PDMS), Polydiphenylsiloxanen (PDPS), Polymethylphenylsiloxanen (PMPS) und/oder Mischungen daraus.,.

2. Polymerisierbare Mischungszusammensetzung nach Anspruch 1, **dadurch gekennzeichnet, dass** die Feststoffkomponente B und die liquide oder halbfeste Komponente A Initiatorkomponenten enthalten.

3. Polymerisierbare Mischungszusammensetzung nach Anspruch 1 oder 2, **dadurch gekennzeichnet, dass** der Anteil der oligomeren oder polymeren Verbindung in der liquiden oder halbfesten Komponente A in einem Bereich zwischen 0,1 und 50 Gew.-%, bevorzugt zwischen 0,1 und 30 Gew.-%, bevorzugt zwischen 0,5 und 25 Gew.-% und besonders bevorzugt zwischen 1 und 20 Gew.-% liegt.

4. Polymerisierbare Mischungszusammensetzung nach Anspruch 1, **dadurch gekennzeichnet, dass** das Polyorganosiloxan mit dem Monomeranteil der liquiden oder halbfesten Komponente A homogen mischbar ist und/oder eine vorzugsweise lineare Kettenstruktur aufweist.

5. Polymerisierbare Mischungszusammensetzung nach Anspruch 4, **dadurch gekennzeichnet, dass** die mittlere Anzahl (n) der linearen Polyorganosiloxangruppen in einem Bereich zwischen 2 und 500, bevorzugt zwischen 2 und 200, bevorzugt zwischen 5 und 200, bevorzugt zwischen 5 und 150, besonders bevorzugt im Bereich zwischen 7 und 75, bevorzugt zwischen 7 und 50 liegt.

6. Polymerisierbare Mischungszusammensetzung nach einem der Ansprüche 4 oder 5, **dadurch gekennzeichnet, dass** die Polyorganosiloxan-Kettenstruktur chemisch modifiziert ist.

7. Polymerisierbare Mischungszusammensetzung nach Anspruch 6, **dadurch gekennzeichnet, dass** die Polyorganosiloxan-Kettenstruktur mittels Substituenten, bevorzugt mittels Polycaprolactonen, besonders bevorzugt ungesättigten Gruppen vorzugsweise (Meth)Arcyl-Gruppen chemisch modifiziert ist.

8. Polymerisierbare Mischungszusammensetzung nach einem der Ansprüche 1-7, **dadurch gekennzeichnet, dass** die polymerisierte Mischungszusammensetzung eine Bruchzähigkeit (Kₘₐₓ) ≥ 1,9 kJ/m^{1/2} und eine Brucharbeit ≥ 900 J/m² aufweist.

9. Verwendung einer polymerisierbaren Mischungszusammensetzung nach einem der Ansprüche 1-7 zur Herstellung eines Kunststoffformkörpers, **dadurch gekennzeichnet, dass** die polymerisierte Mischungszusammensetzung bzw. der Kunststoffformkörper eine Bruchzähigkeit (Kₘₐₓ) ≥ 1,9 kJ/m^{1/2} und eine Brucharbeit ≥ 900 J/m² aufweist.

10. Verwendung nach Anspruch 9, **dadurch gekennzeichnet, dass** die polymerisierbare Mischungszusammensetzung als Reparaturwerkstoff für derartige Formkörper oder dgl. eingesetzt wird.

11. Dentalprothese, hergestellt aus einer polymerisierbaren Mischungszusammensetzung nach mindestens einem der Ansprüche 1-8.

## Claims

1. Polymerizable mixture composition comprising a liquid or semisolid component A with at least one monomer constituent, and one solid component B based on polymethyl methacrylate (PMMA) with a filler and/or additional substances, where the liquid or semisolid component A additionally comprises an oligomeric or polymeric polyorganosiloxane which modifies the monomer constituent and which is miscible with the monomer constituent and is selected from the group consisting of polydimethylsiloxanes (PDMS), polydiphenylsiloxanes (PDPS), polymethylphenylsiloxanes (PMPS) and/or mixtures thereof.

2. Polymerizable mixture composition according to Claim 1, **characterized in that** the solid component B and the liquid or semisolid component A comprise initiator components.

3. Polymerizable mixture composition according to Claim 1 or 2, **characterized in that** the proportion of the oligomeric or polymeric compound in the liquid or semisolid component A is in a range between 0.1 and 50% by weight, preferably between 0.1 and 30% by weight, preferably between 0.5 and 25% by weight and particularly preferably between 1 and 20% by weight.

4. Polymerizable mixture composition according to Claim 1, **characterized in that** the polyorganosiloxane is homogeneously miscible with the monomer constituent of the liquid or semisolid component A and/or has a preferably linear chain structure.

5. Polymerizable mixture composition according to Claim 4, **characterized in that** the average number (n) of the linear polyorganosiloxane groups is in a range between 2 and 500, preferably between 2 and 200, preferably between 5 and 200, preferably between 5 and 150, particularly preferably in the range between 7 and 75, preferably between 7 and 50.

6. Polymerizable mixture composition according to Claim 4 or 5, **characterized in that** the polyorganosiloxane chain structure has been chemically modified.

7. Polymerizable mixture composition according to Claim 6, **characterized in that** the polyorganosiloxane chain structure has been chemically modified by means of substituents, preferably by means of polycaprolactones, particularly preferably by means of unsaturated groups, preferably (meth)acrylic groups.

8. Polymerizable mixture composition according to any of Claims 1-7, **characterized in that** the fracture toughness (Kₘₐₓ) of the polymerized mixture composition is ≥ 1.9 kJ/m^{1/2} and its fracture energy is ≥ 900 J/m².

9. Use of a polymerizable mixture composition according to any of Claims 1-7 for the production of a plastics moulding, **characterized in that** the fracture toughness (Kₘₐₓ) of the polymerized mixture composition and, respectively, of the plastics moulding is ≥ 1.9 kJ/m^{1/2} and its fracture energy is ≥ 900 J/m².

10. Use according to Claim 9, **characterized in that** the polymerizable mixture composition is used as repair material for such mouldings or the like.

11. Dental prosthesis, produced from a polymerizable mixture composition according to at least one of Claims 1-8.

## Revendications

1. Composition de mélange polymérisable contenant un composant liquide ou semi-solide A ayant au moins une proportion de monomère, et un composant solide B à base de polyméthacrylate de méthyle (PMMA) avec une charge et/ou des additifs, le composant liquide ou semi-solide A comprenant en outre au moins un polyorganosiloxane oligomère ou polymère modifiant la proportion de monomère, qui est miscible avec la proportion de monomère, choisi dans le groupe constitué par les polydiméthylsiloxanes (PDMS), les polydiphénylsiloxanes (PDPS), les polyméthylphénylsiloxanes (PMPS) et/ou les mélanges de ceux-ci.

2. Composition de mélange polymérisable selon la revendication 1, **caractérisée en ce que** le composant solide B et le composant liquide ou semi-solide A contiennent des composants initiateurs.

3. Composition de mélange polymérisable selon la revendication 1 ou 2, **caractérisée en ce que** la proportion du composé oligomère ou polymère dans le composant liquide ou semi-solide A se situe dans une plage comprise entre 0,1 et 50 % en poids, de préférence entre 0,1 et 30 % en poids, de préférence entre 0,5 et 25 % en poids, et de manière particulièrement préférée entre 1 et 20 % en poids.

4. Composition de mélange polymérisable selon la revendication 1, **caractérisée en ce que** le polyorganosiloxane est miscible de manière homogène avec la proportion de monomère du composant liquide ou semi-solide A et/ou présente une structure de chaîne de préférence linéaire.

5. Composition de mélange polymérisable selon la revendication 4, **caractérisée en ce que** le nombre moyen (n) des groupes polyorganosiloxane linéaires se situe dans une plage comprise entre 2 et 500, de préférence entre 2 et 200, de préférence entre 5 et 200, de préférence entre 5 et 150, de manière particulièrement préférée dans la plage comprise entre 7 et 75, de préférence entre 7 et 50.

6. Composition de mélange polymérisable selon l'une quelconque des revendications 4 ou 5, **caractérisée en ce que** la structure de chaîne de polyorganosiloxane est modifiée chimiquement.

7. Composition de mélange polymérisable selon la revendication 6, **caractérisée en ce que** la structure de chaîne de polyorganosiloxane est modifiée chimiquement au moyen de substituants, de préférence au moyen de polycaprolactones, de manière particulièrement préférée de groupes insaturés, de préférence de groupes (méth)acryliques.

8. Composition de mélange polymérisable selon l'une quelconque des revendications 1 à 7, **caractérisée en ce que** la composition de mélange polymérisée présente une ténacité à la rupture (Kₘₐₓ) ≥ 1,9 kJ/m^{1/2} et une énergie de rupture ≥ 900 J/m².

9. Utilisation d'une composition de mélange polymérisable selon l'une quelconque des revendications 1 à 7 pour la fabrication d'un corps moulé en matière plastique, **caractérisée en ce que** la composition de mélange polymérisée ou le corps moulé en matière plastique présente une ténacité à la rupture (Kₘₐₓ) ≥ 1,9 kJ/m^{1/2} et une énergie de rupture ≥ 900 J/m².

10. Utilisation selon la revendication 9, **caractérisée en ce que** la composition de mélange polymérisable est utilisée en tant que matériau de réparation pour de tels corps moulés ou analogues.

11. Prothèse dentaire, fabriquée à partir d'une composition de mélange polymérisable selon au moins l'une quelconque des revendications 1 à 8.
